# EUROPEAN PATENT APPLICATION

(11) **EP 4 424 351 A1**
(43) Date of publication of application: **04.09.2024**
(21) Application number: 22897661.9
(22) Date of filing: 15.11.2022
(51) Int. Cl.: A61M 25/09, A61M 25/08, A61M 25/01, A61B 17/00

(54) **MEDICAL CATHETER WIRE DELIVERY MECHANISM AND TRANSLUMINAL INTERVENTION SYSTEM**

(30) Priority: 23.11.2021 CN 202122888450 U
(71) Applicant: CTC Medical Technology (Beijing) Co., Ltd, Beijing 100176 (CN)
(72) Inventor: ZHOU, Haiyan, Beijing 100176 (CN); SHAO, Meng, Beijing 100176 (CN); ZHAO, Lei, Beijing 100176 (CN); HUANG, Haiyun, Beijing 100176 (CN)
(74) Representative: Derry, Paul Stefan
(86) International application number: PCT/CN2022/132066
(87) International publication number: WO 2023/093578

(57) **Abstract**

A medical catheter or guidewire delivery mechanism and a transluminal intervention system are disclosed. The medical catheter or guidewire delivery mechanism comprises: a base (110); a catheter or guidewire (3) comprising a catheter or guidewire proximal end (31) and a catheter or guidewire distal end (32); a catheter or guidewire fixing seat (200), configured to drive the catheter or guidewire proximal end (31) to rotate and fixedly mounted on the base (110); and a catheter or guidewire driving wheel (300), configured to drive the catheter or guidewire distal end (32) to move and fixedly mounted on the base (110), the catheter or guidewire (3) is configured to be in a slack state between the catheter or guidewire fixing seat (200) and the catheter or guidewire driving wheel (300). The catheter or guidewire fixing seat (200) and the catheter or guidewire driving wheel (300) are fixedly arranged on the base (110) at intervals, such an arrangement not only can ensure integration level of individual elements, but also can ensure that the catheter or guidewire (3) always has a certain degree of slackness, moreover, there is no need to regulate a position of the catheter or guidewire proximal end (31) according to a movement of the catheter or guidewire distal end (32), thereby improving operational convenience.

## Description

The present application claims the priority of Chinese Utility Model Patent Application No. 202122888450.7 entitled "MEDICAL CATHETER WIRE DELIVERY MECHANISM AND TRANSLUMINAL INTERVENTION SYSTEM" and filed with China National Intellectual Property Administration on November 23, 2021, the content of which is incorporated herein by reference in its entirety.

### Field of the Invention

The present application belongs to the technical field of interventional therapy, and specifically relates to a medical catheter or guidewire delivery mechanism and a transluminal intervention system.

### Background of the Invention

Interventional therapy is an emerging therapeutic method compromised between a surgical therapy and a medical therapy, and has characteristics of small invasiveness, simplification, safety, effectiveness, fewer complications, and significantly shortened hospital stay time, and thus has received attention from more and more doctors and patients. The interventional therapy is a therapeutic method that is minimally invasive and involves creating a tiny channel with a diameter of several millimeters on a blood vessel or skin, or utilizing a existing conduit in a human body to locally treat lesions under guidance of an imaging device (such as an angiography machine, a fluoroscopy machine, a CT, an MR, and a B-scan ultrasonography machine) without exposing the lesions via a surgery.

In the interventional therapy, a guide wire and a catheter need to be driven to move to the site of the lesions for treatment, and thus the guide wire and the catheter each have a certain length. During the driving process of the guide wire and the catheter, as a distal end of the guide wire and the catheter moves, position of a proximal end of the guide wire and the catheter needs to be adaptively regulated, resulting in that the difficulty of system manipulation is increased.

### Summary of the Invention

### Technical solution

In view of the above defects or deficiencies in the prior art, the present application provides a medical catheter or guidewire delivery mechanism and a transluminal intervention system, each of which has a simple structure and a high integration level, and is easy to operate.

In order to achieve the above objectives, according to one aspect of the present application, a medical catheter or guidewire delivery mechanism is provided, the medical catheter or guidewire delivery mechanism comprises:
a base;
a catheter or guidewire, comprising a catheter or guidewire proximal end and a catheter or guidewire distal end;
a catheter or guidewire fixing seat, configured to drive the catheter or guidewire proximal end to rotate and fixedly mounted on the base; and
a catheter or guidewire driving wheel, configured to drive the catheter or guidewire distal end to move and fixedly mounted on the base,
wherein, the catheter or guidewire is configured to be in a slack state between the catheter or guidewire fixing seat and the catheter or guidewire driving wheel.

In some embodiments, the medical catheter or guidewire delivery mechanism further comprises a guide structure fixedly mounted on the base, and the catheter or guidewire protrudes from the catheter or guidewire fixing seat and extends around a plate surface of the guide structure to the catheter or guidewire driving wheel.

In some embodiments, on the base, the catheter or guidewire fixing seat and the catheter or guidewire driving wheel are located on a same side of the guide structure.

In some embodiments, on the base, the catheter or guidewire fixing seat is spaced apart from and arranged perpendicular to the catheter or guidewire driving wheel; or, on the base, the catheter or guidewire fixing seat is spaced apart from and arranged parallel to the catheter or guidewire driving wheel.

In some embodiments, the guide structure is in an open shape, and comprises a bottom plate protruding from the base and configured to support the catheter or guidewire, and two limit plates arranged on the bottom plate at intervals; and the limit plates are configured to limit a movement range of the catheter or guidewire in a width direction of the bottom plate.

In some embodiments, a top surface of the bottom plate is formed with a flat guide portion for coiling the catheter or guidewire; or, the top surface of the bottom plate is formed with a convex guide portion for coiling the catheter or guidewire.

In some embodiments, the guide structure is a sleeve component, and comprises a guide shell and a limiting accommodation space that is surrounded and defined by the guide shell and configured to accommodate the catheter or guidewire.

In some embodiments, the catheter or guidewire fixing seat comprises:
a rotation driving motor, configured to drive the catheter or guidewire to rotate and mounted on the base; and
a catheter or guidewire clamp, configured to clamp and fix the catheter or guidewire proximal end and rotatably mounted on the base;
wherein, the rotation driving motor is connected to the catheter or guidewire clamp in a transmission manner.

In some embodiments, the catheter or guidewire driving wheel comprises a catheter or guidewire main drive wheel for outputting a rotation driving force and an idler wheel, and the catheter or guidewire is sandwiched between the catheter or guidewire main drive wheel and the idler wheel.

In addition, the present application also provides a luminal intervention system, which comprises the medical catheter or guidewire delivery mechanism as described above.

### Advantageous effects

According to the medical catheter or guidewire delivery mechanism and the luminal intervention system of the present application, the medical catheter or guidewire delivery mechanism adopts a cooperated structure of the catheter or guidewire fixing seat, the catheter or guidewire driving wheel and the guide structure, wherein the catheter or guidewire fixing seat and the catheter or guidewire driving wheel are fixed on the base at intervals, such an arrangement not only can ensure integration level of individual elements, but also can ensure that the catheter or guidewire always has a certain degree of slackness, moreover, there is no need to regulate a position of the catheter or guidewire proximal end according to a movement of the catheter or guidewire distal end, thereby improving operational convenience. The guide structure can support the catheter or guidewire, which can ensure that the catheter or guidewire always fits the guide structure, and can regulate a deflection angle of the catheter or guidewire, so as to maintain a certain radian of the catheter or guidewire and ensure order movement of the catheter or guidewire.

### Brief Description of the Drawings

The accompanying drawings are configured to provide an understanding of the present application and form a part of the specification. They are configured together with the specific embodiments below to explain the present application, but do not constitute a limitation to the present application, in which:
Fig. 1 is a schematic view of a structure of a medical catheter wire delivery mechanism according to a specific embodiment of the present application;
Fig. 2 is a schematic view of another structure of a medical catheter or guidewire delivery mechanism according to a specific embodiment of the present application;
Fig. 3 is a view of the structure of Fig. 2 at a different viewing perspective, in which a base and a guide structure are shown;
Fig. 4 is a schematic view of a local structure of Fig. 2, in which a guide structure and a catheter wire are shown;
Fig. 5 is a schematic view of the structure of Fig. 4 at a different viewing perspective, in which a guide structure and a catheter or guidewire are shown; and
Fig. 6 is a schematic diagram of another structure of a guide structure of a medical catheter or guidewire delivery mechanism according to a specific embodiment of the present application.

### Description of Reference Numerals

| | | | |
|---|---|---|---|
| 110 | Base | 3 | Catheter or guidewire |
| 31 | Catheter or guidewire proximal end | 32 | Catheter or guidewire distal end |
| 200 | Catheter or guidewire fixing seat | 300 | Catheter or guidewire driving wheel |
| 400 | Guide structure | | |
| 41 | Bottom plate | 42 | Limit plates |
| 1 | Support block | | |
| 101 | Bottom surface of support block | 102 | Top surface of support block |
| 210 | Rotation driving motor | 220 | Catheter or guidewire clip |
| 310 | Catheter or guidewire main drive wheel | 320 | Idler wheel |

### Detailed Description of the Embodiments

Hereafter, the specific embodiments of the present application will be illustrated in detail in combination with the accompanying drawings. It should be understood that, the specific embodiments described here are only used to illustrate and explain the present application, but are not intended to limit the present application.

It should be noted that, when there is no conflict, the embodiments and the features in the embodiments of the present application may be combined with each other.

In the present application, unless otherwise illustrated, the orientation words such as "upper, lower, top, and bottom" that are used are usually serve as the terms for describing a mutual position relationship of individual components in a direction as shown in the drawings or in a vertical, perpendicular, or gravity direction.

In the interventional therapy, a guide wire and a catheter need to be driven to move to the site of lesions for treatment, and thus the guide wire and the catheter each have a certain length. During the driving process of the guide wire and the catheter, as a distal end of the guide wire and the catheter moves, position of a proximal end of the guide wire and the catheter needs to be adaptively regulated, resulting in that the difficulty of system manipulation is increased. Therefore, how to improve the convenience of the system operations becomes a problem deserved to be studied. The specific structures and functions of the medical catheter or guidewire delivery mechanism and the luminal intervention system will be set forth in detail below.

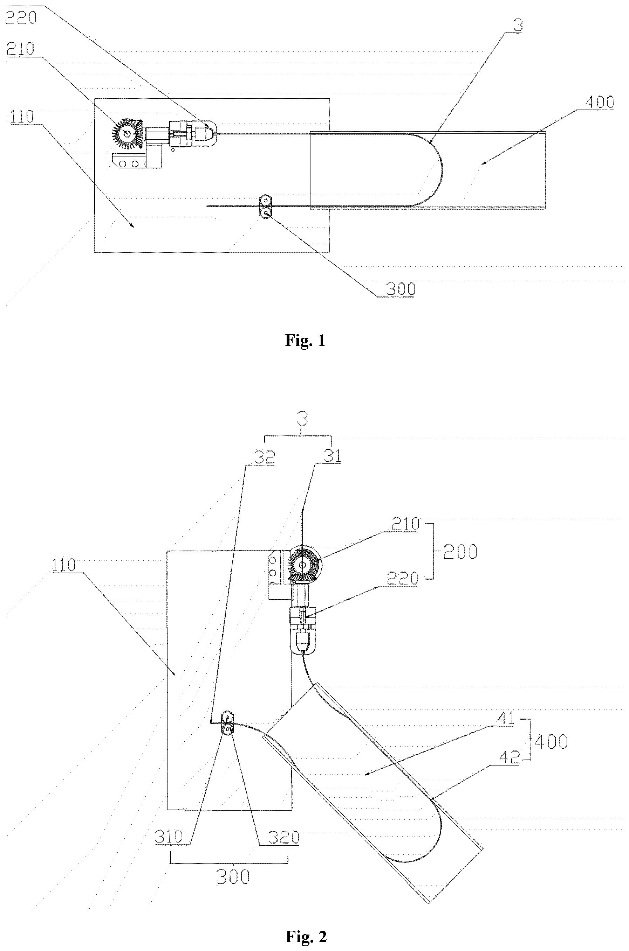

Referring to Figs. 1 to 6, the present application discloses a medical catheter or guidewire delivery mechanism, which comprises: a base 110, a catheter or guidewire 3, a catheter or guidewire fixing seat 200, and a catheter or guidewire driving wheel 300. It should be noted that, with regard to various medical guide wires or catheters, they are collectively referred to as catheter or guidewire below.

Specifically, the base 110, serving as a mounting body in the medical catheter or guidewire delivery mechanism, is configured for mounting and fixation of other components, such that other components in the medical catheter or guidewire delivery mechanism are integrated on the base 110, thereby improving integration level of the medical catheter or guidewire delivery mechanism.

The catheter or guidewire 3 comprises a catheter or guidewire proximal end 31 and a catheter or guidewire distal end 32. The catheter or guidewire proximal end 31 and the catheter or guidewire distal end 32 are located at two ends of the catheter or guidewire 3, respectively. The catheter or guidewire distal end 32 may be moved to the site of lesions and perform corresponding treatment on the lesions.

The catheter or guidewire fixing seat 200 is configured to drive the catheter or guidewire proximal end 31 to rotate and fixedly mounted on the base 110. The catheter or guidewire fixing seat 200 can drive the catheter or guidewire proximal end 31 to rotate, and in turn to drive the catheter or guidewire distal end 32 for direction regulation.

And, the catheter or guidewire drive wheel 300 is configured to drive the catheter or guidewire distal end 32 to move and fixedly mounted on the base 110. The catheter or guidewire drive wheel 300 can drive the catheter or guidewire distal end 32 to move to reach the site of lesions.

Wherein, the catheter or guidewire 3 is configured to be in a slack state between the catheter or guidewire fixing seat 200 and the catheter or guidewire driving wheel 300. It should be noted that, with regard to the slack state, it may be understood, that there is no tension between the catheter or guidewire distal end 32 and the catheter or guidewire proximal end 31, and the catheter or guidewire 3 is not subjected to a pulling force in a length direction of the catheter or guidewire 3. The catheter or guidewire fixing seat 200 and the catheter or guidewire driving wheel 300 are fixedly arranged on the base 110 at intervals, such an arrangement not only can ensure integration level of individual elements, but also can ensure that the catheter or guidewire 3 always has a certain degree of slackness, moreover, there is no need to regulate a position of the catheter or guidewire proximal end 31 according to a movement of the catheter or guidewire distal end 32, thereby improving operational convenience.

Optionally, the base 110 may have a flat plate-like structure, wherein the various components of the medical catheter or guidewire delivery mechanism may be mounted on multiple surfaces of the base 110 or may be mounted on one surface of the base 110, such an arrangement reduces the processing and material cost of the base 110, reduces a self-weight of the medical catheter or guidewire delivery mechanism, thereby facilitating operations of the medical personnel. The base 110 may be diverse in shape; and it may be a rectangular plate that is convenient for processing, or an L-shaped plate that is easy to grip, or may be in other irregular shapes, without specific limitations.

Optionally, with regard to the catheter or guidewire 3, a guide wire, a catheter, a micro-guide wire, a micro-catheter, or a balloon catheter, etc., may be used in the interventional surgery. The catheter or guidewire 3 is a conventional catheter or guidewire in the prior art, and may be one or more of the aforementioned catheters or guidewires without specific limitations.

Optionally, the catheter or guidewire fixing seat 200 may comprise a driving element that is mounted on the base 110, and a fixing element that is mounted on the driving element and can fix the catheter or guidewire 3. In this way, when the driving element rotates, the catheter or guidewire 3 is driven to rotate by the fixing element, so as to regulate a movement direction of the catheter or guidewire distal end 32. Of course, the catheter or guidewire fixing seat 200 is not limited thereto, as long as it can fix the catheter or guidewire proximal end 31 and drive it to rotate.

Optionally, the catheter or guidewire driving wheel 300 may comprise a driving element that is mounted on the base 110, and a catheter or guidewire pushing wheel set that can push the catheter or guidewire 3 to move. The catheter or guidewire 3 may be sandwiched within the catheter or guidewire pushing wheel set. When the driving element rotates, it can drive the catheter or guidewire pushing wheel set to rotate, at this time, the catheter or guidewire 3 that contacts with the catheter or guidewire pushing wheel set can move along a rotation direction of the catheter or guidewire pushing wheel set, thereby achieving position movement regulation of the catheter or guidewire distal end 32.

Optionally, with regard to the mounting positions of the catheter or guidewire fixing seat 200 and the catheter or guidewire driving wheel 300, both of them may be arranged on the base 110 at intervals, wherein, the catheter or guidewire fixing seat 200 and the catheter or guidewire driving wheel 300 may be arranged parallelly or may be arranged to form a included angle therebetween, such an arrangement can ensure that the catheter or guidewire 3 passing through the catheter or guidewire fixing seat 200 and the catheter or guidewire driving wheel 300 forms a curved section structure, so as to ensure that catheter or guidewire 3 always maintains in a slack state, moreover, the catheter or guidewire distal end 32 may be driven for position regulation without moving the catheter or guidewire proximal end 31, thereby improving operational convenience and achieving high integration level.

With regard to the specific structure of the medical catheter or guidewire delivery mechanism, in an embodiment, the medical catheter or guidewire delivery mechanism further comprises a guide structure 400 fixedly mounted on the base 110, and the catheter or guidewire 3 protrudes from the catheter or guidewire fixing seat 200 and extends around a plate surface of the guide structure 400 to the catheter or guidewire driving wheel 300, as shown in Figs. 1 and 2. In a movement direction of the catheter or guidewire 3, the guide structure 400 is located between the catheter or guidewire fixing seat 200 and the catheter or guidewire driving wheel 300, and can support the catheter or guidewire 3 and ensure that the catheter or guidewire 3 always fits the guide structure 400, and regulate a deflection angle of the catheter or guidewire 3, such that the catheter or guidewire 3 maintains a certain radian, i.e. in a slack state, thereby ensuring order movement of the catheter or guidewire 3.

Further, in an embodiment, on the base 110, the catheter or guidewire fixing seat 200 and the catheter or guidewire driving wheel 300 are located on a same side of the guide structure 400, as shown in Figure 1. It may be understood that, the catheter or guidewire fixing seat 200, the catheter or guidewire driving wheel 300, and the guide structure 400 are mounted on the base 110; the guide structure 400 may comprise multiple surfaces, and the catheter or guidewire fixing seat 200 and the catheter or guidewire driving wheel 300 are on the same side of the guide structure 400, without being separated by the guide structure 400. In this way, a curved arrangement of the catheter or guidewire may be achieved. For example, the guide structure 400 comprises a left plate end surface, a right plate end surface, an upper plate end surface, and a lower plate end surface, as shown in Figure 1. Both of the catheter or guidewire fixing seat 200 and the catheter or guidewire driving wheel 300 are located on the left side of the left plate end surface.

Further, with regard to the positional relationship between the catheter or guidewire fixing seat 200 and the catheter or guidewire driving wheel 300, in an embodiment, on the base 110, the catheter or guidewire fixing seat 200 is spaced apart from and arranged perpendicular to the catheter or guidewire driving wheel 300, as shown in Figure 2, such that the catheter or guidewire 3 is in a U shape with an external opening at the ends, thereby ensuring that the catheter or guidewire 3 always fits the guide structure 400 along an inner contour of the guide structure 400.

In another embodiment, on the base 110, the catheter or guidewire fixing seat 200 is spaced apart from and arranged parallel to the catheter or guidewire driving wheel 300, as shown in Figure 1, such that the catheter or guidewire is in a U shape, thereby further improving integration level of the medical catheter or guidewire delivery mechanism, and ensuring that the catheter or guidewire 3 always fits the guide structure 400.

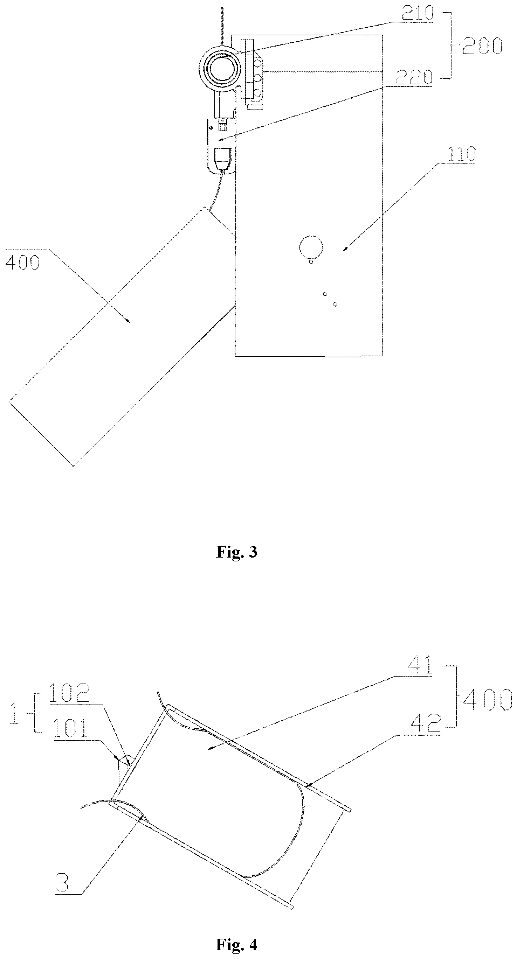

With regard to a specific structure of the guide structure 400, in an embodiment, the guide structure 400 is in an open shape, and comprises a bottom plate 41 protruding from the base 110 and configured to support the catheter or guidewire 3, and two limit plates 42 arranged on the bottom plate 41 at intervals; and the limit plates 42 are configured to limit a movement range of the catheter or guidewire 3 in a width direction of the bottom plate 41. As shown in Figs. 3 and 4, it may be understood that, the catheter or guidewire 3 located on the guide structure 400 comprises two straight sections that fit the limit plates 42 and a natural arc-shaped section that is connected between the two straight sections. A spacing between the two straight sections may be regulated and defined by means of the limit plates 42, thereby improving integration level of the catheter or guidewire 3 and ensuring order movement of the catheter or guidewire 3.

In addition, with regard to a connection structure between the guide structure and the base 110, the bottom plate 41 may directly adhere onto the base 110 and may also be mounted onto the base 110 via a mounting block. In this way, the mounting block not only can regulate a height of a catheter or guidewire supporting surface of the bottom plate 41 and also can regulate a deflection angle of the catheter or guidewire supporting surface relative to the base 110. For example, when an output end of the catheter or guidewire fixing seat 200 and an input end of the catheter or guidewire driving wheel 300 are not in the same plane, by means of the mounting block, the catheter or guidewire supporting surface may be in an inclination state gradually transiting from the output end of the catheter or guidewire fixing seat 200 to the input end of the catheter or guidewire driving wheel 300, thereby ensuring natural transition of catheter or guidewire 3.

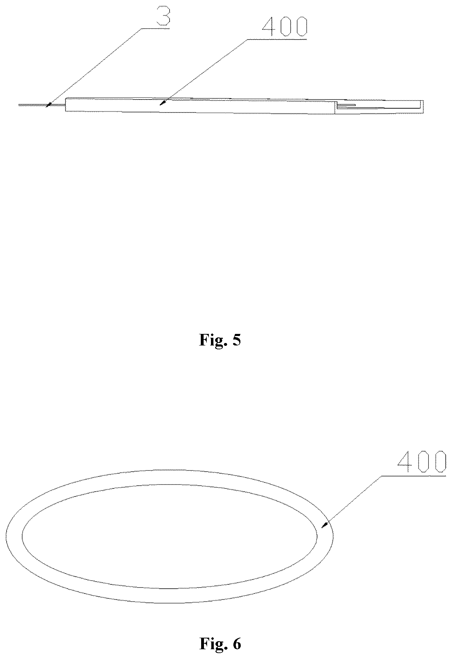

Further, the surface of the bottom plate 41 for supporting the catheter or guidewire 3 is designed, such that, in an embodiment, a top surface of the bottom plate 41 is formed with a flat guide portion for coiling the catheter or guidewire 3, as shown in Figure 5. It may be understood that, the bottom plate 41 includes a top surface for supporting the catheter or guidewire 3, which may be a smooth surface, such that the catheter or guidewire 3 will always move smoothly, thereby ensuring order movement of the catheter or guidewire 3.

In another embodiment, the top surface of the bottom plate 41 is formed with a convex guide portion for coiling the catheter or guidewire 3, which is not shown in the figure. It may be understood that, the top surface may be in a convex shape in a length direction, and thus an area of the top surface thereof may be increased when a length of the bottom plate 41 is constant, such that a longer catheter or guidewires 3 may be conveniently supported, thereby facilitating integrated arrangement of the medical catheter or guidewire delivery mechanism.

In addition, with regard to the guide structure 400, in an embodiment, the guide structure 400 is a sleeve component, and comprises a guide shell and a limiting accommodation space that is surrounded and defined by the guide shell and configured to accommodate the catheter or guidewire, as shown in Fig. 6. At this point, the catheter or guidewire supporting surface of the guide structure 400 is in a concave shape, and the limiting accommodation space may effectively limit the catheter or guidewire 3, so as to prevent the catheter or guidewire 3 falling off from the guide shell.

Meanwhile, with regard to other components of the medical catheter or guidewire delivery mechanism, in an embodiment, the catheter or guidewire fixing seat 200 comprises: a rotation driving motor 210, configured to drive the catheter or guidewire 3 to rotate and mounted on the base 110; and a catheter or guidewire clamp 220, configured to clamp and fix the catheter or guidewire proximal end 31 and rotatably mounted on the base 110; wherein, the rotation driving motor 210 is connected to the catheter or guidewire clamp 220 in a transmission manner. As shown in Figs. 1 and 3, the base 110 may comprise a front surface and a back surface, wherein, the rotation driving motor 210 may be mounted on the back surface of the base 110 and protrude out of the front surface of the base 110, and the catheter or guidewire clamp 220 is located on the front surface of the base 110, such an arrangement improves integration level of the mounted individual components and avoids that excessive components are concentrated on the front surface of the base 110, thereby ensuring order movement of the catheter or guidewire 3. A supporting seat for the catheter or guidewire clamp 220 may be mounted on the base 110, the catheter or guidewire clamp 220 can self-rotate on the supporting seat, and the rotation driving motor 210 can drive the catheter or guidewire clamp 220 to rotate, so as to drive the catheter or guidewire 3 to rotate, thereby achieving a turning movement of the catheter or guidewire distal end 32. It should be noted that, the rotation driving motor 210 may be a commercially available small- or micro-sized driving motor, without specific limitations herein.

With regard to the catheter or guidewire driving wheel 300, in an embodiment, the catheter or guidewire driving wheel 300 comprises a catheter or guidewire main drive wheel 310 for outputting a rotation driving force and an idler wheel 320, and the catheter or guidewire 3 is sandwiched between the catheter or guidewire main drive wheel 310 and the idler wheel 320. As shown in Fig. 2, the catheter or guidewire main drive wheel 310 of the catheter or guidewire driving wheel 300 can actively rotate, drive the catheter or guidewire 3 to move and drive the idler wheel 320 to rotate, so as to stably drive the catheter or guidewire 3 forward or backward.

In addition, the present application further provides a luminal intervention system, which comprises the medical catheter or guidewire delivery mechanism as described above, wherein the catheter or guidewire proximal end 31 of the catheter or guidewire 3 is fixed on the catheter or guidewire clamp 220, and the catheter or guidewire distal end 32 may be sandwiched between the catheter or guidewire main drive wheel 310 and the idler wheel 320. The catheter or guidewire driving wheel 300 drives the catheter or guidewire 3 to move, and the catheter wire fixing seat 200 drives the catheter or guidewire 3 to rotate to regulate the deflection angle of the catheter or guidewire distal end 32, such that the catheter or guidewire distal end 32 can reach the site of lesions for treatment.

It should be noted that, the other components and functions of the medical catheter or guidewire delivery mechanism and the transluminal intervention system according to the embodiments of the present application are known to those skilled in the art, and redundant descriptions thereof are omitted for conciseness.

The preferred embodiments of the present application are described in detail above in combination with the accompanying drawings. However, the present application is not limited to the specific details of the above embodiments, and various simple modifications (such as changes in the shape, thickness, and material of an end plate sealing layer) may be made to the technical solutions of the present application within the scope of the technical concept of the present application, all of which fall within the protection scope of the present application.

In addition, it should be noted that, in the description of the present specification, the description with reference to terms such as "one embodiment", "some embodiments", "example", "specific example", or "some examples" etc. means that the specific features, structures, materials, or characteristics described in combination with the embodiment or example are contained in at least one embodiment or example of the present application. In the present specification, the schematic expressions of the above terms are not necessarily directed at the same embodiment or example. Moreover, the specific features, structures, materials, or characteristics that are described may be combined in any one or more of the embodiments or examples in an appropriate manner. If there is no contradiction, they may be combined in any appropriate manner. In order to avoid unnecessary repetitions, various possible combinations will not be otherwise illustrated in the present application.

In addition, various different embodiments of the present application may also be combined arbitrarily, as long as the combinations will not depart from the idea of the present application, and they should also be regarded as the content disclosed in the present application.

## Claims

1. A medical catheter or guidewire delivery mechanism, comprising:
a base (110);
a catheter or guidewire (3), comprising a catheter or guidewire proximal end (31) and a catheter or guidewire distal end (32);
a catheter or guidewire fixing seat (200), configured to drive the catheter or guidewire proximal end (31) to rotate and fixedly mounted on the base (110); and
a catheter or guidewire driving wheel (300), configured to drive the catheter or guidewire distal end (32) to move and fixedly mounted on the base (110),
wherein, the catheter or guidewire (3) is configured to be in a slack state between the catheter or guidewire fixing seat (200) and the catheter or guidewire driving wheel (300).

2. The medical catheter or guidewire delivery mechanism of claim 1, wherein, the medical catheter or guidewire delivery mechanism further comprises a guide structure (400) fixedly mounted on the base (110), and the catheter or guidewire (3) protrudes from the catheter or guidewire fixing seat (200) and extends around a plate surface of the guide structure (400) to the catheter or guidewire driving wheel (300).

3. The medical catheter or guidewire delivery mechanism of claim 2, wherein, on the base (110), the catheter or guidewire fixing seat (200) and the catheter or guidewire driving wheel (300) are located on a same side of the guide structure (400).

4. The medical catheter or guidewire delivery mechanism of claim 3, wherein, on the base (110), the catheter or guidewire fixing seat (200) is spaced apart from and arranged perpendicular to the catheter or guidewire driving wheel (300); or, on the base (110), the catheter or guidewire fixing seat (200) is spaced apart from and arranged parallel to the catheter or guidewire driving wheel (300).

5. The medical catheter or guidewire delivery mechanism of claim 2, wherein, the guide structure (400) is in an open shape, and comprises a bottom plate (41) protruding from the base (110) and configured to support the catheter or guidewire (3), and two limit plates (42) arranged on the bottom plate (41) at intervals; and the limit plates (42) are configured to limit a movement range of the catheter or guidewire (3) in a width direction of the bottom plate (41).

6. The medical catheter or guidewire delivery mechanism of claim 5, wherein, a top surface of the bottom plate (41) is formed with a flat guide portion for coiling the catheter or guidewire (3); or, the top surface of the bottom plate (41) is formed with a convex guide portion for coiling the catheter or guidewire (3).

7. The medical catheter or guidewire delivery mechanism of claim 2, wherein, the guide structure (400) is a sleeve component, and comprises a guide shell and a limiting accommodation space that is surrounded and defined by the guide shell and configured to accommodate the catheter or guidewire.

8. The medical catheter or guidewire delivery mechanism of claim 1, wherein, the catheter or guidewire fixing seat (200) comprises:
a rotation driving motor (210), configured to drive the catheter or guidewire (3) to rotate and mounted on the base (110); and
a catheter or guidewire clamp (220), configured to clamp and fix the catheter or guidewire proximal end (31) and rotatably mounted on the base (110);
wherein, the rotation driving motor (210) is connected to the catheter or guidewire clamp (220) in a transmission manner.

9. The medical catheter or guidewire delivery mechanism of claim 1, wherein, the catheter or guidewire driving wheel (300) comprises a catheter or guidewire drive wheel (310) for outputting a rotation driving force and an idler wheel (320), and the catheter or guidewire (3) is sandwiched between the catheter or guidewire drive wheel (310) and the idler wheel (320).

10. A luminal intervention system, comprising a medical catheter or guidewire delivery mechanism, wherein the medical catheter or guidewire delivery mechanism comprises:
a base (110);
a catheter or guidewire (3), comprising a catheter or guidewire proximal end (31) and a catheter or guidewire distal end (32);
a catheter or guidewire fixing seat (200), configured to drive the catheter or guidewire proximal end (31) to rotate and fixedly mounted on the base (110); and
a catheter or guidewire driving wheel (300), configured to drive the catheter or guidewire distal end (32) to move and fixedly mounted on the base (110),
wherein, the catheter or guidewire (3) is configured to be in a slack state between the catheter or guidewire fixing seat (200) and the catheter or guidewire driving wheel (300).

11. The medical catheter or guidewire delivery mechanism of claim 10, wherein, the medical catheter or guidewire delivery mechanism further comprises a guide structure (400) fixedly mounted on the base (110), and the catheter or guidewire (3) protrudes from the catheter or guidewire fixing seat (200) and extends around a plate surface of the guide structure (400) to the catheter or guidewire driving wheel (300).

12. The medical catheter or guidewire delivery mechanism of claim 11, wherein, on the base (110), the catheter or guidewire fixing seat (200) and the catheter or guidewire driving wheel (300) are located on a same side of the guide structure (400).

13. The medical catheter or guidewire delivery mechanism of claim 12, wherein, on the base (110), the catheter or guidewire fixing seat (200) is spaced apart from and arranged perpendicular to the catheter or guidewire driving wheel (300); or, on the base (110), the catheter or guidewire fixing seat (200) is spaced apart from and arranged parallel to the catheter or guidewire driving wheel (300).

14. The medical catheter or guidewire delivery mechanism of claim 11, wherein, the guide structure (400) is in an open shape, and comprises a bottom plate (41) protruding from the base (110) and configured to support the catheter or guidewire (3), and two limit plates (42) arranged on the bottom plate (41) at intervals, and the limit plates (42) are configured to limit a movement range of the catheter or guidewire (3) in a width direction of the bottom plate (41).

15. The medical catheter or guidewire delivery mechanism of claim 14, wherein, a top surface of the bottom plate (41) is formed with a flat guide portion for coiling the catheter or guidewire (3); or, the top surface of the bottom plate (41) is formed with a convex guide portion for coiling the catheter or guidewire (3).

16. The medical catheter or guidewire delivery mechanism of claim 11, wherein, the guide structure (400) is a sleeve component, and comprises a guide shell and a limiting accommodation space that is surrounded and defined by the guide shell and configured to accommodate the catheter or guidewire.

17. The medical catheter or guidewire delivery mechanism of claim 10, wherein, the catheter or guidewire fixing seat (200) comprises:
a rotation driving motor (210), configured to drive the catheter or guidewire (3) to rotate and mounted on the base (110); and
a catheter or guidewire clamp (220), configured to clamp and fix the catheter or guidewire proximal end (31) and rotatably mounted on the base (110);
wherein, the rotation driving motor (210) is connected to the catheter or guidewire clamp (220) in a transmission manner.

18. The medical catheter or guidewire delivery mechanism of claim 10, wherein, the catheter or guidewire driving wheel (300) comprises a catheter or guidewire drive wheel (310) for outputting a rotation driving force and an idler wheel (320), and the catheter or guidewire (3) is sandwiched between the catheter or guidewire drive wheel (310) and the idler wheel (320).
